(19) Europäisches Patentamt
European Patent Office
Office européen des brevets

(11) Publication number: 0 123 449 B1

# (12) EUROPEAN PATENT SPECIFICATION

(45) Date of publication of patent specification: 03.05.89

(51) Int. Cl.[4]: C 07 C 1/20, C 07 C 11/02 // B01J29/28

(21) Application number: 84302027.2

(22) Date of filing: 27.03.84

## (54) Process for converting alcohols/ethers into olefins using steamed zeolite catalyst.

(30) Priority: 22.04.83 US 487549

(43) Date of publication of application: 31.10.84 Bulletin 84/44

(45) Publication of the grant of the patent: 03.05.89 Bulletin 89/18

(84) Designated Contracting States: BE DE FR GB IT NL

(56) References cited:
DE-A-2 935 863
US-A-4 083 889
US-A-4 311 865
US-A-4 326 994
US-A-4 359 595

The file contains technical information submitted after the application was filed and not included in this specification

(73) Proprietor: MOBIL OIL CORPORATION
150 East 42nd Street
New York New York 10017 (US)

(72) Inventor: Chang, Clarence Dayton
11 Murray Place
Princeton, NJ 08540 (US)
Inventor: Chu, Cynthia Ting-Wah
283 Westcott Boulevard
Pennington, NJ 08534 (US)

(74) Representative: Jones, Alan John et al
CARPMAELS & RANSFORD 43 Bloomsbury Square
London, WC1A 2RA (GB)

EP 0 123 449 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the manufacture of light olefin hydrocarbons from lower alcohols and/or their ethers.

A remarkable growth in the production of synthetic fibers, plastics and rubber has taken place in recent decades. This growth has been supported and encouraged to a large extent by an expanding supply of inexpensive petrochemical raw materials such as ethylene, propylene and four and five carbon atom olefins. Side by side with this remarkable development there has been an increasing demand for alkylate, made by reacting olefins with isobutane, for use as a high octane gasoline component. Environmental factors which limit the lead content of gasoline are likely to increase the need for alkylate and for other high-octane gasoline blending stocks.

Burgeoning demand for olefins, particularly ethylene, propylene and butenes, has of course led to periods of shortage, due either to short supply of suitable feedstocks or to limited processing capacity. In any case, it would appear desirable to provide efficient means for converting raw materials other than petroleum into olefins and/or high octane gasoline.

The production of olefinic materials from alcohols and/or their corresponding ethers over crystalline aluminosilicate zeolite catalysts has been described in many patent publications, for example U.S. Patents 3,244,766; 3,931,349; 4,025,576; 4,052,479; 4,058,576 and 4,083,889.

Processes for converting lower alcohols and ethers into olefins generally utilize a fixed bed of catalyst. The feedstock containing, for example methanol and dimethyl ether, is introduced at one end of the reaction zone in which the conversion to light hydrocarbons is effected, and an effluent stream containing the desired light olefins is removed from the bed.

US Patent 4,359,595 describes such a process in which the catalyst comprises a steamed crystalline aluminosilicate of crystal size less than 1 µm; that process, however, is carried out in such a way as to produce a largely aromatics' product. US Patent 4,311,865 describes a process that does achieve a predominantly lower olefin product, but using a catalyst containing not only steamed crystalline aluminosilicate of alpha value 10 to 60, but also cobalt which does, of course, contribute its own catalytic function.

The present invention is based on the observation that certain zeolites having a crystal size of less than 1 µm and which have been steamed to reduce their alpha values have a dramatically better activity for converting alcohols/ethers into olefins compared to unsteamed zeolites of equivalent alpha values.

In accordance with the invention, there is provided a process for producing a hydrocarbon mixture rich in olefins which comprises contacting a feedstock comprising an alcohol or ether or mixture thereof with a crystalline aluminosilicate zeolite, characterized in that the crystalline aluminosilicate zeolite has a crystal size of less than 1 µm and has been steamed prior to contact with the feedstock to reduce its alpha value to from 5 to 35.

The alcohols and their ether derivatives used as starting materials for the process may be manufactured from synthesis gas (a mixture of CO and $H_2$ made from coal or from natural gas), they may be produced by fermentation, or they may be manufactured from petroleum fractions. The olefin hydrocarbons produced by the process may in turn be converted into alkylate or into aromatics and blended with gasoline, or they may be separated and used as petrochemicals. The invention therefore provides a novel means for producing hydrocarbon petrochemicals and fuels from raw materials other than petroleum.

The mixture of olefins produced by the process contains mostly ethylene, propylene and the butylenes with a small pentenes component and this is so regardless of whether the starting material is methanol, dimethyl ether, ethanol or other alcohols or ethers. The process therefore clearly differs from classical dehydration in which the olefin produced bears a simple relation to the alcohol starting material.

It is a noteworthy feature of this invention that highly desirable hydrocarbon by-products are formed along with the olefins. In particular, gasoline boiling range constituents comprising $C_{5+}$ paraffin, olefins, naphthenes and aromatics, including pentanes, pentenes, and higher boiling materials, are formed in greater or less quantity, depending on the particular reaction conditions chosen. Substantially all of these products boil at a temperature below about 215°C so that a gasoline boiling range by-product may be recovered when producing olefins by this process. This gasoline by-product tends to be rich in aromatic hydrocarbons and isoparaffins, and is therefore characterized by a high octane number. When recovered, the $C_{5+}$ to 215°C fraction may be used directly as gasoline or as a high octane gasoline blending stock.

The olefins formed in the process of the invention can be used in a number of different ways, including conversion into gasoline or into gasoline plus distillate, for example by the techniques described in U.S. Patents 4,025,576 and 4,227,992.

The production of olefins by the catalytic conversion of lower alcohols utilizing zeolitic catalysts primarily of the ZSM-5 type is well known in the art. It is also known in the art that in general, olefin production is enhanced by operating at less severe operating conditions, including lower catalytic activity whereas aromatics' production is favored at conditions of increased severity. In this connection, German Application P 29 35 863.2 describes the conversion of methanol over high silica-to-alumina ratio ZSM-5 type materials which would inherently have low alpha values.

In contrast, the surprising observation on which the present invention is based is that the production of olefins from an alcohol feed is greatly

enhanced by the use of a zeolite catalyst having a certain crystal size and which has been steamed to reduce its alpha activity from a high value to a lower value.

The lower alcohols/ethers that may be used as starting materials in the process of the invention include methanol, ethanol, propanol and isopropanol and/or their corresponding ether derivatives. The feed may consist of a relatively pure single alcohol, or mixtures of these alcohols with other components such as higher alcohols. In general, any mixture comprising methanol or ethanol or propanol or isopropanol may be used.

The preferred starting materials are ethanol and methanol. Particularly preferred are mixtures comprising more than 25 weight percent of methanol, especially mixtures of methanol and dimethyl ether.

The degree of zeolite catalyst activity for all acid catalyzed reaction can be expressed in terms of "alpha value". The alpha value reflects the relative activity of the catalyst with respect to a high activity silica-alumina cracking catalyst. To determine the alpha value as that term is used herein, n-hexane conversion is determined at a suitable temperature between about 290 and 540°C, preferably at 540°C. Conversion is varied by variation in space velocity such that a conversion of up to about 60 percent of n-hexane is obtained to convert to a rate constant per unit volume of zeolite and compared with that of silica-alumina catalyst which is normalized to a reference activity of 540°C. Activities of the catalyst are expressed as multiples of the silica-alumina standard. The silica-alumina reference catalyst contains about 10 percent $Al_2O_3$ and the remainder $SiO_2$. This method of determining alpha, modified as described above, is more fully described in the Journal of Catalysis, Vol. VI, pp. 278—287, 1966.

It is important to note that the alpha values thus determined are not a direct measurement of the activity of the zeolite for the conversion of alcohols and ethers into olefinic hydrocarbons and, in fact, one of the surprising discoveries on which the invention is based is that materials which have the same alpha value behave differently and another surprising discovery is that steaming enhances the activity of certain zeolites that have a crystal size of less than 1 µm whereas steaming does just the opposite to zeolites that have a crystal size greater than 1 µm. Thus, although it is true that the zeolite must be steam treated to reduce its alpha value to a lower value, the simple fact remains that the zeolite must be of the proper crystal size in order to achieve a catalyst of enhanced activity.

The techniques of steaming zeolites to obtain desired alpha values are well known in the art.

When the treating atmosphere is steam, the temperature may be from 260°C to 985°C depending on the steam pressure, with the use of higher pressures requiring lower temperatures. This treatment is carried on for a period of time sufficient to effect the desired reduction in alpha. Generally, such periods will be from about 0.5 to 100 hours. A treating atmosphere may be employed which is 100 percent steam or steam mixed with a gas which is substantially inert with respect to the zeolite. The treatment will generally be carried out at atmospheric pressure, but pressures ranging from sub-atmospheric to several hundred atmospheres may be employed. With the use of elevated pressures, temperatures in the lower region of the above range will usually be applicable in achieving the desired reduction in alpha value of the zeolite under treatment. Thus, it has been found that at elevated steam pressure, the temperature of treatment can be reduced substantially to achieve the same degree of modification.

The preferred crystalline zeolites for use in the process of the invention include ZSM-5, ZSM-11, ZSM-12, ZSM-23, ZSM-35, ZSM-38 and ZSM-48, with ZSM-5 being particularly preferred.

It may be useful to incorporate the crystalline zeolite into a matrix comprising another material resistant to the temperature and other conditions employed in the process. Such a matrix material is useful as a binder and imparts greater resistance to the catalyst towards the severe temperature, pressure and reactant feed stream velocity conditions encountered in the process.

Useful matrix materials include both synthetic and naturally occurring substances, especially inorganic materials such as clays, silica and/or metal oxides.

The relative proportions of zeolite component and matrix material, on an anhydrous basis, may vary widely with the zeolite content ranging from 1 to 99 percent by weight and more usually from 5 to 80 percent by weight of the dry composite.

It should be understood that when the zeolitic materials are incorporated into a matrix, the resulting composite can have a particle diameter greater than 1 µm. What is necessary in carrying out the process of the invention is that the zeolite itself which is incorporated into the matrix has a crystal size of less than 1 µm.

Since the conversion of methanol can be satisfactorily described by a simple kinetic model and can be expressed as

$$A \xrightarrow{k_1} B \xrightarrow{k_2} C$$

where A=oxygenates, B=olefins, C=paraffins+aromatics, and rate constants $k_1$ and $k_2$ are associated with olefin formation and aromatics' formation, respectively, a convenient comparison of the ability of different catalysts to convert methanol into olefins and aromatics can be obtained by comparing the respective rate constants for such different catalysts. Such comparisons are described in the following Example which illustrates the invention.

Example

Reaction studies were conducted in a 9.5 mm stainless steel microreactor with zeolite catalysts in the steamed and unsteamed condition. Tests

were conducted as atmospheric pressure and LHSV of 1.0 to 300 and the feedstock was pure methanol. The temperature was 500°C. In all cases the catalyst was a hydrogen-exchanged ZSM-5 zeolite.

In some cases the ZSM-5 was not steamed whereas in other cases the ZSM-5 was steamed at 538°C for 16 hours to 140 hours at 1 atmosphere of steam.

Both small crystal size (less than 1 µm) and large crystal size (greater than 1 µm) ZSM-5 were evaluated and the results are graphically depicted in Figures 1, 2 and 3 over a range of alpha values.

The alpha value of the ZSM-5 before steaming ranged from 174—340 alpha for the small crystal size (less than 1 µm) and was 163 alpha for the large crystal (greater than 1 µm).

As can be seen from Figure 1 of the accompanying drawings, which is a plot of alpha value against $k_1$, steaming enhances the olefin formation rate ($k_1$) of small crystal ZSM-5 only.

Figure 2, which is a plot of alpha value against $k_2$, demonstrates that steaming enhances the aromatization rate ($k_2$) of both small and large crystal ZSM-5.

Figure 3, which is a plot of alpha value against $k_1/k_2$, demonstrates that olefin selectivity ($k_1/k_2$) is enhanced by the use of small crystal ZSM-5.

## Claims

1. A process for producing a hydrocarbon mixture rich in olefins which comprises contacting a feedstock comprising an alcohol and/or ether with a crystalline aluminosilicate zeolite, characterized in that the crystalline aluminosilicate zeolite has a crystal size of less than 1 µm and has been steamed prior to contact with the feedstock to reduce its alpha value to from 5 to 35.

2. A process according to claim 1 wherein the feedstock is methanol.

3. A process according to claim 1 or claim 2 wherein the zeolite is ZSM-5, ZSM-11, ZSM-12, ZSM-23, ZSM-35, ZSM-38 or ZSM-48.

4. A process according to any of claims 1 to 3 wherein the zeolite is the hydrogen form of ZSM-5.

5. A process according to any preceding claim wherein the zeolite is incorporated in a matrix.

## Patentansprüche

1. Verfahren zur Herstellung einer an Olefinen reichen Kohlenwasserstoffmischung, bei dem das Zufuhrmaterial, das einen Alkohol und/oder Ether umfaßt, mit einem kristallinen Aluminosilicatzeolith in Kontakt gebracht wird, dadurch gekennzeichnet, daß der kristalline Aluminosilicatzeolith eine Kristallgröße von kleiner als 1 µm aufweist und vor dem Kontakt mit dem Zufuhrmaterial mit Dampf behandelt wurde, um seinen α-Wert auf von 5 bis 35 zu verringern.

2. Verfahren nach Anspruch 1, worin das Zufuhrmaterial Methanol ist.

3. Verfahren nach Anspruch 1 oder 2, worin der Zeolith ZSM-5, ZSM-11, ZSM-12, ZSM-23, ZSM-35, ZSM-38 oder ZSM-48 ist.

4. Verfahren nach einem der Ansprüche 1 bis 3, worin der Zeolith die Wasserstofform von ZSM-5 ist.

5. Verfahren nach einem der vorstehenden Ansprüche, worin der Zeolith in eine Matrix eingearbeitet ist.

## Revendications

1. Un procédé de production d'un mélange d'hydrocarbures riche en oléfines qui consiste à mettre une charge comprenant un alcool et/ou un éther au contact d'une zéolite à base d'un aluminosilicate cristallin, ce procédé étant caractérisé en ce que la zéolite à base d'aluminosilicates cristallins présente une dimension cristalline inférieure à 1 µm et a été soumise à un traitement à la vapeur préalablement au contact avec la charge, pour que sa valeur passe de 5 à 35.

2. Un procédé selon la revendication 1, dans laquelle la charge consiste en méthanol.

3. Un procédé selon la revendication 1 ou la revendication 2, dans lequel la zéolite consiste en ZSM-5, ZSM-11, ZSM-12, ZSM-23, ZSM-35, ZSM-38 ou ZSM-48.

4. Un procédé selon l'une quelconque des revendications 1 à 3, dans lequel la zéolite est la forme protonée de la ZSM-5.

5. Un procédé selon l'une quelconque des revendications précédentes dans lequel la zéolite est incorporée à un support.

Fig.1

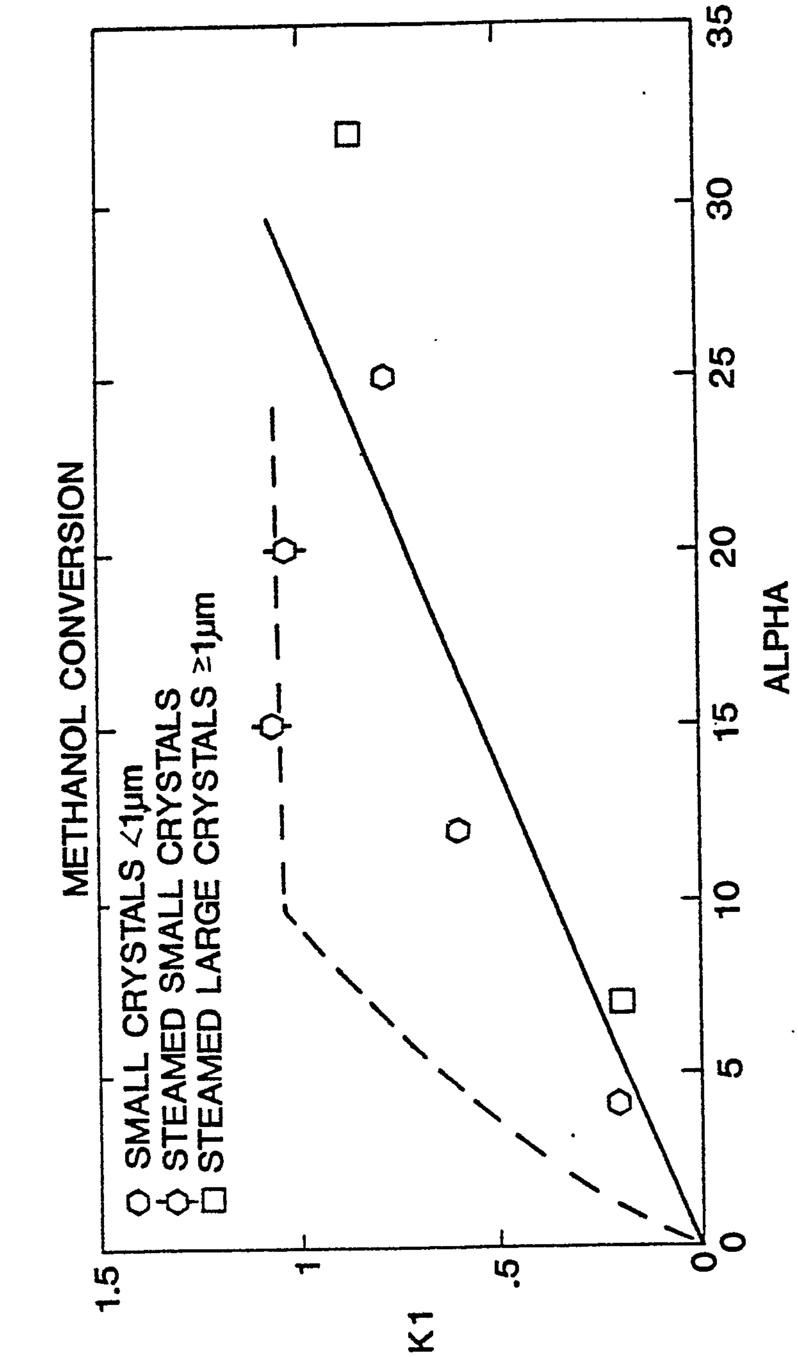
METHANOL CONVERSION
SMALL CRYSTALS ∠1µm
STEAMED SMALL CRYSTALS
STEAMED LARGE CRYSTALS ≥1µm
K1
1.5
1
.5
0
0
5
10
15
20
25
30
35
ALPHA

Fig. 2

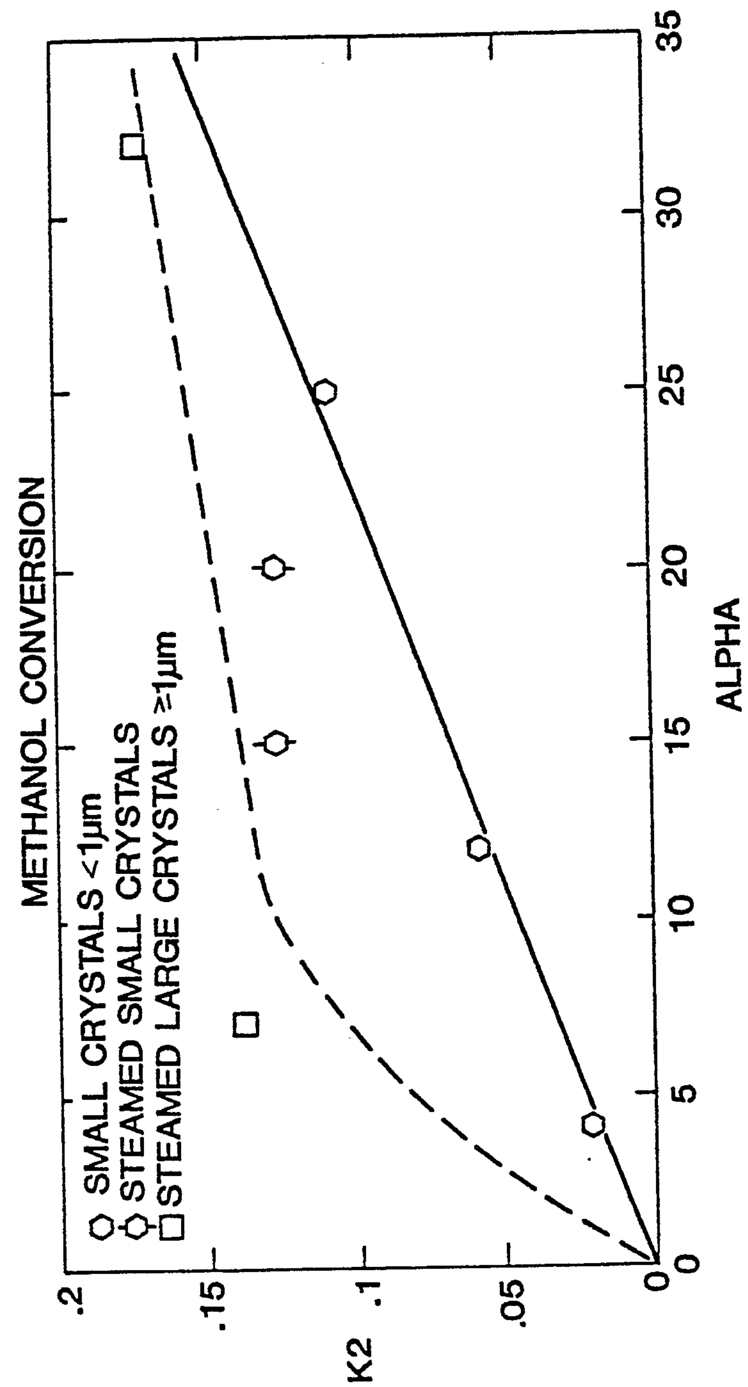
METHANOL CONVERSION
SMALL CRYSTALS <1μm
STEAMED SMALL CRYSTALS
STEAMED LARGE CRYSTALS ≥1μm
K2
0
.05
.1
.15
.2
0
5
10
15
20
25
30
35
ALPHA

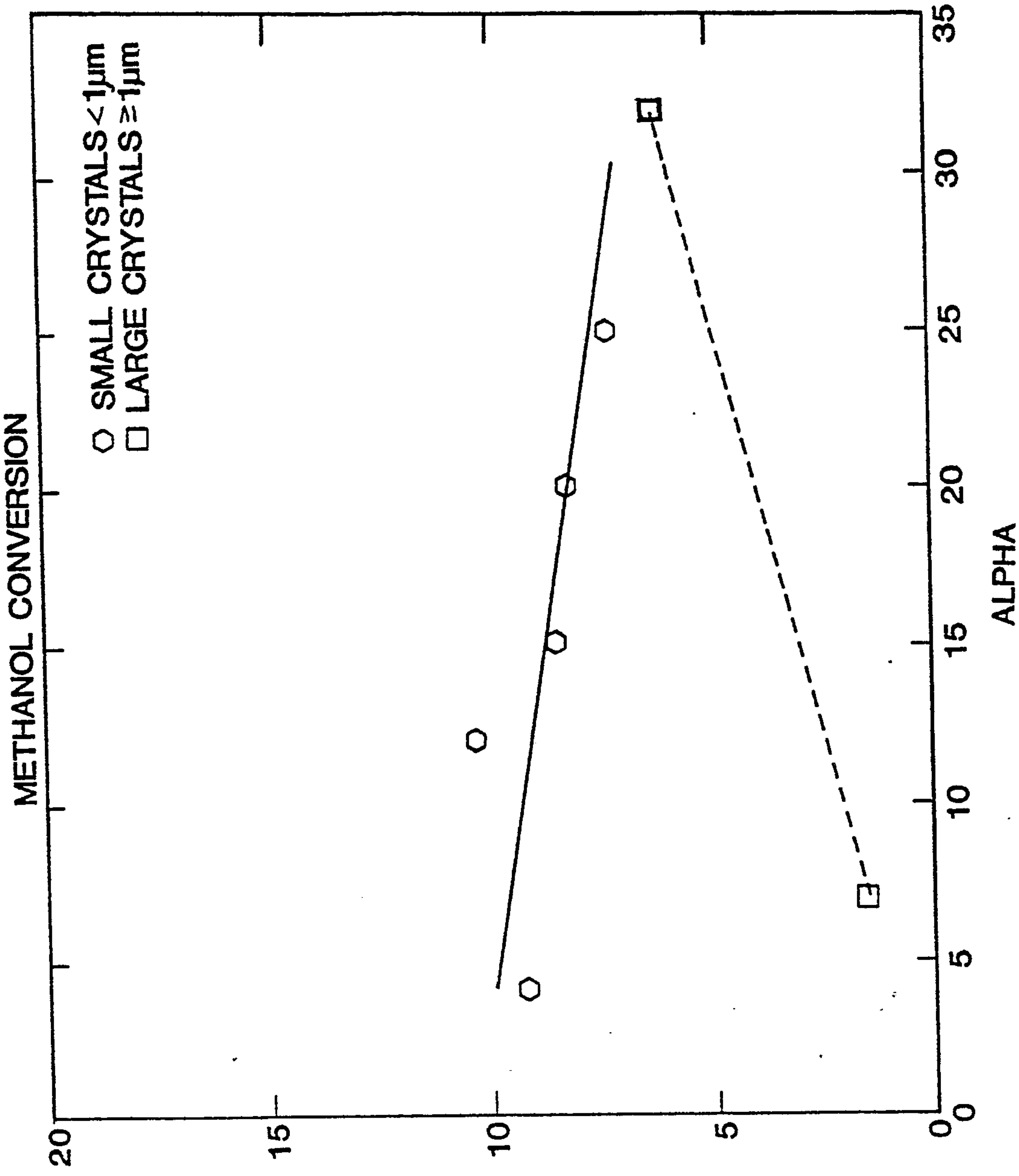
METHANOL CONVERSION
○ SMALL CRYSTALS<1μm
□ LARGE CRYSTALS≥1μm
K1/K2
0
5
10
15
20
ALPHA
0
5
10
15
20
25
30
35

Fig. 3